# EUROPEAN PATENT SPECIFICATION

(11) **EP 4 312 925 B1**
(45) Date of publication and mention of the grant of the patent: **19.03.2025**
(21) Application number: 22715866.4
(22) Date of filing: 21.03.2022
(51) Int. Cl.: A61F 13/01, A61B 46/00, A61F 13/15

(54) **ABSORBENT COMPOSITES**
ABSORBIERENDE VERBUNDWERKSTOFFE
COMPOSITES ABSORBANTS

(30) Priority: 22.03.2021 US 202163164217 P
(43) Date of publication of application: 07.02.2024
(73) Proprietor: Berry Global, Inc., Evansville, Indiana 47710 (US)
(72) Inventor: WANG, Lei, Mooresville, North Carolina 28117 (US); FU, Linlei, Suzhou, Jiangsu 215100 (CN); JIN, Yongji, Shanghai 200082 (CN)
(74) Representative: Grünecker Patent- und Rechtsanwälte PartG mbB
(86) International application number: PCT/US2022/021218
(87) International publication number: WO 2022/204066

(56) References cited:
- US-A1- 2016 206 393
- US-A1- 2019 021 913

## Description

### CROSS-REFERENCE TO RELATED APPLICATIONS

This application claims priority under 35 U.S.C. §119 to U.S. Patent Application No. 63/164,217 filed March 22, 2021.

### TECHNICAL FIELD

Embodiments of the presently-disclosed invention relate generally to absorbent composites including a nonwoven top layer, an absorbent core including a plurality of individual layers bonded together, and optionally a polymeric film.

### BACKGROUND

Absorbent materials are typically positioned around the fenestration area (e.g., a window through which a surgical procedure may be performed) in a surgical drape. The absorbent material is located around the fenestration area to capture a limited amount of fluid generated during a surgical intervention. These fluids can include body fluids (e.g., blood) and/or fluids used by the surgical team in the performance of their work.

Typically, such absorbent materials comprise cellulosic fibers such as rayon and lyocell in a spunlaced or/and chemical bonded web. Such fabrics have good absorption capacity, however, they suffer from high run off and high absorption time. These two deficiencies indicate that the fabric is slow to take up fluid, which can be detrimental during surgery as it may allow fluid to flow out of the area covered with the absorbent material.

A different group of absorbent materials contain wood pulp to provide absorption. This raw material (e.g., wood pulp), however, has a tendency to form lint in the form of particulates released by the fabric. The creation of lint during a surgical procedure is not desirable.

Another class of absorbent material currently used consists of a three-layer composite including a cover made from a spunbond, a core made from hydrophilic meltblown, and a backing film. This type of fabric, however, exhibits a lower absorption capacity (g/g) while still suffering from high run off.

In yet another class of absorbent materials, a reasonably bulky hydrophilic spunbond is been laminated to a film. Such products, however, also suffers from poor run off (i.e., the % run off is high) and lower absorbency than the cellulosic containing absorbent materials.

US2019/021913 discloses an absorbent composite with a nonwoven top layer and an absorbent core.

Therefore, there remains a need in the art for a cost effective absorbent material, such as for use around a fenestration area on a surgical drape, as a tray cover, or in the healthcare industry for the prevention and/or treatment of skin breakdown in a patient, that has one or more of good abrasion resistance, fast rate of absorption to avoid leakage as characterized by low absorption time, low percentage of run off, and high absorbency.

### SUMMARY OF INVENTION

One or more embodiments of the invention may address one or more of the aforementioned problems. The invention provides an absorbent composite including a nonwoven top layer, an absorbent core directly or indirectly attached to the nonwoven top layer, and an optional film layer. The nonwoven top layer comprises a plurality of hydrophilic fibers, in which the fibers may be rendered hydrophilic via topical application of a hydrophilic additive and/or via addition of a hydrophilic additive to the polymer melt used to form at least some (or all) of the fibers forming the nonwoven top layer. The nonwoven top layer may comprise a generally open structure to allow relatively fast penetration by fluids. The absorbent composite comprises an absorbent core including a plurality of layers including (i) at least one physically entangled nonwoven layer (e.g., a hydroentangled layer), and (ii) at least one bonded carded nonwoven layer and/or at least one thermal bonded high-loft nonwoven layer (e.g., a hydrophilic high-loft nonwoven layer that has been thermally consolidated). Similar to the nonwoven top layer, the absorbent core may also comprise a plurality of hydrophilic fibers, in which the fibers may be rendered hydrophilic via topical application of a hydrophilic additive and/or via addition of a hydrophilic additive to the polymer melt used to form at least some (or all) of the fibers forming the absorbent core layer. For example, the at least one physically entangled nonwoven and/or the at least one bonded carded nonwoven web may comprise a plurality of hydrophilic fibers, in which the fibers may be rendered hydrophilic via topical application of a hydrophilic additive and/or via addition of a hydrophilic additive to the polymer melt used to form at least some (or all) of the fibers thereof. Additionally or alternatively, the at least one physically entangled nonwoven and/or the at least one bonded carded nonwoven web may comprise cellulosic fibers (e.g., fibers formed from regenerated cellulose). The absorbent composite has (i) a composite-rate of absorption of less than 8 seconds for a 5 ml liquid sample as determined by D824-94, and (ii) a composite-run-off value of less than 15% as determined by ISO 9073-11. In accordance with certain embodiments of the invention, the absorbent composite may optionally include a film directly or indirectly attached (e.g., bonded) to the absorbent core, such that the absorbent core is directly or indirectly sandwiched between the nonwoven top layer and the film.

In another aspect, the invention provides a surgical gown, surgical drape, a portion of a surgical drape, or a tray liner comprising an absorbent composite as disclosed herein. In accordance with certain embodiments of the invention, the absorbent composite comprises a fenestration material surrounding a fenestration (e.g., window or aperture) through which a surgical procedure can be performed.

In another not claimed aspect, the invention provides a material suitable for use, for example alone or when incorporated into an article of manufacture, in the healthcare industry for the prevention and/or treatment of skin breakdown, which can undesirably lead to complications such as decubitus ulcers. A patient, for example, may experience skin breakdown at or during several points throughout the care of a patient in a hospital, a nursing home, or a homecare setting. In accordance with certain embodiments not claimed of the invention, for instance, an absorbent composite either alone or as part of an article of manufacture (e.g., adult diaper, bedding sheet, gown, etc.) that may be placed in contact with the skin of a patient. In this regard, certain embodiments not according to the invention also provide methods of preventing skin deterioration (e.g., decubitus ulcers) of an individual susceptible to development of skin deterioration (e.g., decubitus ulcers). Individuals to susceptible to development of skin deterioration may include any patient that may spend a considerable amount of time one or a few positions (e.g., a patient that is mostly or wholly confined to a bed) over the course of multiple days. In this regard, absorbent composites may provide a micro-climate environment at or adjacent the skin of an individual having one or more of a desirable air permeability, a low coefficient of friction, and/or highly absorbent for proper humidity levels. In another aspect, certain embodiments not according to the invention also provide methods of treating individuals already suffering or showing signs of skin deterioration (e.g., decubitus ulcers). In this regard, the absorbent may provide a micro-climate environment (as noted above) at or adjacent the skin of the individual already suffering or showing signs of skin deterioration (e.g., decubitus ulcers) such that the rate or severity of the skin deterioration may be positively impacted (e.g., rate of deterioration may be slowed, stopped, and/or reversed).

In another aspect, the invention provides a method of making an absorbent composite including the following steps: (i) providing a nonwoven top layer comprising hydrophilic fibers;
(ii) providing an absorbent core, in which the absorbent core comprises a plurality of layers including at least one physically entangled nonwoven layer and at least one bonded carded nonwoven layer; and (iii) directly or indirectly attaching the nonwoven top layer and the absorbent core to provide the absorbent composite as disclosed described herein. In accordance with certain embodiments of the invention, the method may further comprise topically treating the nonwoven top layer, the absorbent core, or both with a hydrophilic additive. Methods, in accordance with certain embodiments of the invention, may further comprise attaching a film directly or indirectly to the absorbent core, wherein the absorbent core layer is directly or indirectly sandwiched between the nonwoven top layer and the film.

### BRIEF DESCRIPTION OF THE DRAWING(S)

The invention now will be described more fully hereinafter with reference to the accompanying drawings, in which some, but not all embodiments of the invention are shown. Indeed, this invention may be embodied in many different forms and should not be construed as limited to the embodiments set forth herein; rather, these embodiments are provided so that this disclosure will satisfy applicable legal requirements. Like numbers refer to like elements throughout, and wherein:
Figure 1 illustrates an absorbent composite according to certain embodiments of the invention;
Figure 2 illustrates an absorbent composite including an absorbent core having two physically entangled nonwoven layers according to certain embodiments of the invention;
Figure 3 illustrates an absorbent composite including an absorbent core having two bonded carded nonwoven layers located between two physically entangled nonwoven layers according to certain embodiments of the invention;
Figure 4 illustrates an absorbent composite including a film layer bonded to the absorbent core according to certain embodiments of the invention;
Figure 5 illustrates an absorbent composite including a film layer adhesively bonded to the absorbent core by and adhesive layer according to certain embodiments of the invention;
Figure 6 illustrates a surgical drape including an absorbent composite disposed around a fenestration.

### DETAILED DESCRIPTION

The invention now will be described more fully hereinafter with reference to the accompanying drawings, in which some, but not all embodiments of the invention are shown. Indeed, this invention may be embodied in many different forms and should not be construed as limited to the embodiments set forth herein; rather, these embodiments are provided so that this disclosure will satisfy applicable legal requirements. As used in the specification, and in the appended claims, the singular forms "a", "an", "the", include plural referents unless the context clearly dictates otherwise.

This invention provides an absorbent composite including a nonwoven top layer, in which the nonwoven top layer comprises a plurality of hydrophilic fibers (e.g., rendered hydrophilic through the selection of its melt formulation for forming the fibers and/or through topical treatment), and an absorbent core directly or indirectly attached to the nonwoven top layer, in which the absorbent core comprises a plurality of individual nonwoven layers bonded together and including at least one physically entangled nonwoven layer and at least one bonded carded web nonwoven layer and/or at least one thermal bonded high-loft nonwoven layer (e.g., a hydrophilic high-loft nonwoven layer that has been thermally consolidated). Similar to the nonwoven top layer, the absorbent core may also comprise a plurality of hydrophilic fibers (e.g., rendered hydrophilic through the selection of its melt formulation for forming the fibers and/or through topical treatment). In accordance with certain embodiments of the invention, the nonwoven top layer may comprise a pre-bonded spunmelt nonwoven (e.g., spunbond, meltblown, or combinations thereof), such as a point bonded calendered cover, or a carded web made from staple fibers that have been bonded, such as thermally or ultrasonically point bonded. The nonwoven top layer may be attached to the absorbent core via one or more bonding means, such as by thermal point bonding, ultrasonic bonding (e.g., ultrasonic point bonding), and/or adhesive bonding (e.g., adhesively glued together). Absorbent composites, in accordance with certain embodiments of the invention, may provide a variety of particularly desirable properties for an absorbent composite, such as a reduced absorption rate and/or reduced run-off rate while maintaining desirable levels of absorption capacity and/or absorption amount per weight of the absorbent composite. For instance, the improved absorption rate and run off percentages may be realized without negatively impacting other properties (e.g., absorption capacity and/or absorption amount per weight of the absorbent composite) in accordance with certain embodiments of the invention. The absorbent composite has (i) a composite-rate of absorption of less than 8 seconds for a 5 ml liquid sample as determined by D824-94, and (ii) a composite-run-off value of less than 15% as determined by ISO 9073-11. In accordance with certain embodiments of the invention, the absorbent composite may optionally include a film directly or indirectly attached (e.g., bonded) to the absorbent core, such that the absorbent core is directly or indirectly sandwiched between the nonwoven top layer and the film.

In accordance with certain embodiments of the invention, the nonwoven top layer may provide good hydrophilicity, good openness (e.g., porosity), and good abrasion resistance while the absorbent core may provide a high void volume. In accordance with certain embodiments of the invention, the absorbent core may comprise continuous and/or staple monocomponent and/or continuous and/or staple multicomponent (e.g., bicomponent) fibers (e.g., non-crimped, crimped, and/or thermally-crimpable fibers). In accordance with certain embodiments of the invention, the absorbent core may comprise at least one physically entangled nonwoven layer (e.g., hydroentangled) having a blend of continuous and/or staple monocomponent fibers. Additionally or alternatively, the at least one physically entangled nonwoven layer (e.g., hydroentangled) may have a blend of continuous and/or staple bicomponent fibers (e.g., non-crimped, crimped, and/or thermally-crimpable fibers) selected to provide resiliency as well as a desirable pore structure as evaluated by air permeability. Additionally or alternatively, the at least one physically entangled nonwoven layer (e.g., hydroentangled) may comprise a plurality of apertures extending through the entire thickness thereof to facilitate intake of liquid. Additionally or alternatively, the at least one physically entangled nonwoven layer (e.g., hydroentangled) may comprise a plurality of cellulosic fibers either alone or in combination with synthetic fibers, which may be rendered hydrophilic. In accordance with certain embodiments of the invention. the absorbent core may also comprise at least on bonded carded nonwoven layer. The at least on bonded carded nonwoven layer may comprise a plurality of staple fibers (e.g., non-crimped, crimped, and/or thermally-crimpable fibers), which may also be rendered hydrophilic. In accordance with certain embodiments of the invention, at least one physically entangled nonwoven layer is positioned between the at least one bonded carded nonwoven layer and the top nonwoven layer. In accordance with certain embodiments of the invention, the absorbent core may be devoid of pulp and/or meltblown fibers.

The terms "substantial" or "substantially" may encompass the whole amount as specified, according to certain embodiments of the invention, or largely but not the whole amount specified according to other embodiments of the invention.

The terms "polymer" or "polymeric", as used interchangeably herein, may comprise homopolymers, copolymers, such as, for example, block, graft, random, and alternating copolymers, terpolymers, etc., and blends and modifications thereof. Furthermore, unless otherwise specifically limited, the term "polymer" or "polymeric" shall include all possible structural isomers; stereoisomers including, without limitation, geometric isomers, optical isomers or enantionmers; and/or any chiral molecular configuration of such polymer or polymeric material. These configurations include, but are not limited to, isotactic, syndiotactic, and atactic configurations of such polymer or polymeric material. The term "polymer" or "polymeric" shall also include polymers made from various catalyst systems including, without limitation, the Ziegler-Natta catalyst system and the metallocene/single-site catalyst system. The term "polymer" or "polymeric" shall also include, in according to certain embodiments of the invention, polymers produced by fermentation process or biosourced.

The term "nonwoven", as used herein, may comprise a web having a structure of individual fibers, filaments, and/or threads that are interlaid but not in an identifiable repeating manner as in a knitted or woven fabric. Nonwoven fabrics or webs, according to certain embodiments of the invention, may be formed by any process conventionally known in the art such as, for example, meltblowing processes, spunbonding processes, hydroentangling, air-laid, and bonded carded web processes.

The term "staple fiber", as used herein, may comprise a cut fiber from a filament. In accordance with certain embodiments, any type of filament material may be used to form staple fibers. For example, staple fibers may be formed from cellulosic fibers, polymeric fibers, and/or elastomeric fibers. Examples of materials may comprise cotton, rayon, wool, nylon, polypropylene, and polyethylene terephthalate. The average length of staple fibers may comprise, by way of example only, from about 2 centimeter to about 15 centimeter.

The term "continuous fiber", as used herein, may comprise a filament that has a high length-to-diameter aspect ratio (i.e., length : diameter) such as, for example, exceeding about 500,000 : 1, exceeding about 750,000 : 1, or exceeding about 1,000,000 : 1. In accordance with certain embodiments of the invention, the term "continuous "fiber" may comprise a filament that is essentially endless in length.

The term "spunbond", as used herein, may comprise fibers which are formed by extruding molten thermoplastic material as filaments from a plurality of fine, usually circular, capillaries of a spinneret with the diameter of the extruded filaments then being rapidly reduced. According to an embodiment of the invention, spunbond fibers are generally not tacky when they are deposited onto a collecting surface and may be generally continuous. It is noted that the spunbond used in certain composites of the invention may include a nonwoven described in the literature as SPINLACE^{®}.

The term "meltblown", as used herein, may comprise fibers formed by extruding a molten thermoplastic material through a plurality of fine die capillaries as molten threads or filaments into converging high velocity, usually hot, gas (e.g. air) streams which attenuate the filaments of molten thermoplastic material to reduce their diameter, which may be to microfiber diameter, according to certain embodiments of the invention. According to an embodiment of the invention, the die capillaries may be circular. Thereafter, the meltblown fibers are carried by the high velocity gas stream and are deposited on a collecting surface to form a web of randomly disbursed meltblown fibers. Meltblown fibers are microfibers which may be continuous or discontinuous and are generally tacky when deposited onto a collecting surface.

The term "layer", as used herein, may comprise a generally recognizable combination of similar material types and/or functions existing in the X-Y plane.

The term "bicomponent fibers", as used herein, may comprise fibers formed from at least two different polymers extruded from separate extruders but spun together to form one fiber. Bicomponent fibers are also sometimes referred to as conjugate fibers or multicomponent fibers. The polymers are arranged in a substantially constant position in distinct zones across the cross-section of the bicomponent fibers and extend continuously along the length of the bicomponent fibers. The configuration of such a bicomponent fiber may be, for example, a sheath-and-core arrangement wherein one polymer is surrounded by another, or may be a side-by-side arrangement, a pie arrangement, or an "islands-in-the-sea" arrangement, each as is known in the art of multicomponent, including bicomponent, fibers. The "bicomponent fibers" may be thermoplastic fibers that comprise a core fiber made from one polymer that is encased within a thermoplastic sheath made from a different polymer or have a side-by-side arrangement of different thermoplastic fibers. The first polymer often melts at a different, typically lower, temperature than the second polymer. In the sheath/core arrangement, these bicomponent fibers provide thermal bonding due to melting of the sheath polymer, while retaining the desirable strength characteristics of the core polymer. In the side-by-side arrangement, the fibers shrink and crimp creating z-direction expansion.

The term "crimp" or "crimped", as used herein, may comprise a two- or three-dimensional curl or bend such as, for example, a folded or compressed portion having an "L" configuration, a wave portion having a "zig-zag" configuration, or a curl portion such as a helical configuration. In accordance with certain embodiments of the invention, the term "crimp" or "crimped" does not include random two-dimensional waves or undulations in a fiber, such as those associated with normal lay-down of fibers in a melt-spinning process.

The term "high-loft", as used herein, may comprises a material that is compressible by 20% or more when an applied pressure changes from 0.1kPa to 0.5kPa according to BS EN ISO 9703-2 (1995). Moreover, "high-loft" nonwovens, as used herein, may comprise a z-direction thickness generally in excess of about 3 mm and a relatively low bulk density. The thickness of a "high-loft" nonwoven layer may be greater than 3 mm (e.g., greater than 4 mm or greater than 5 mm) as determined according to ASTM D573-95, ITS 120.2 . "High-loft" nonwovens, as used herein, may additionally have a relatively low density (e.g., bulk density - weight per unit volume), such as less than about 50 kg/m³.

All whole number end points disclosed herein that can create a smaller range within a given range disclosed herein are within the scope of certain embodiments of the invention. By way of example, a disclosure of from about 10 to about 15 includes the disclosure of intermediate ranges, for example, of: from about 10 to about 11; from about 10 to about 12; from about 13 to about 15; from about 14 to about 15; etc. Moreover, all single decimal (e.g., numbers reported to the nearest tenth) end points that can create a smaller range within a given range disclosed herein are within the scope of certain embodiments of the invention. By way of example, a disclosure of from about 1.5 to about 2.0 includes the disclosure of intermediate ranges, for example, of: from about 1.5 to about 1.6; from about 1.5 to about 1.7; from about 1.7 to about 1.8; etc.

In accordance with certain embodiments, the present invention provides an absorbent composite including a nonwoven top layer, an absorbent core directly or indirectly attached to the nonwoven top layer, and an optional film layer. The nonwoven top layer comprises a plurality of hydrophilic fibers, in which the fibers may be rendered hydrophilic via topical application of a hydrophilic additive and/or via addition of a hydrophilic additive to the polymer melt used to form at least some (or all) of the fibers forming the nonwoven top layer. The nonwoven top layer may comprise a generally open structure to allow relatively fast penetration by fluids. The absorbent composite comprises an absorbent core including a plurality of layers including (i) at least one physically entangled nonwoven layer (e.g., a hydroentangled layer), and (ii) at least one bonded carded nonwoven layer and/or at least one thermal bonded high-loft nonwoven layer (e.g., a hydrophilic high-loft nonwoven layer that has been thermally consolidated). Similar to the nonwoven top layer, the absorbent core may also comprise a plurality of hydrophilic fibers, in which the fibers may be rendered hydrophilic via topical application of a hydrophilic additive and/or via addition of a hydrophilic additive to the polymer melt used to form at least some (or all) of the fibers forming the absorbent core layer. For example, the at least one physically entangled nonwoven and/or the at least one bonded carded nonwoven web may comprise a plurality of hydrophilic fibers, in which the fibers may be rendered hydrophilic via topical application of a hydrophilic additive and/or via addition of a hydrophilic additive to the polymer melt used to form at least some (or all) of the fibers thereof. Additionally or alternatively, the at least one physically entangled nonwoven and/or the at least one bonded carded nonwoven web may comprise cellulosic fibers (e.g., fibers formed from regenerated cellulose). In accordance with certain embodiments of the invention, the absorbent composite has (i) a composite-rate of absorption of less than 8 seconds for a 5 ml liquid sample as determined by D824-94, and (ii) a composite-run-off value of less than 15% as determined by ISO 9073-11. In accordance with certain embodiments of the invention, the absorbent composite may optionally include a film directly or indirectly attached (e.g., bonded) to the absorbent core, such that the absorbent core is directly or indirectly sandwiched between the nonwoven top layer and the film.

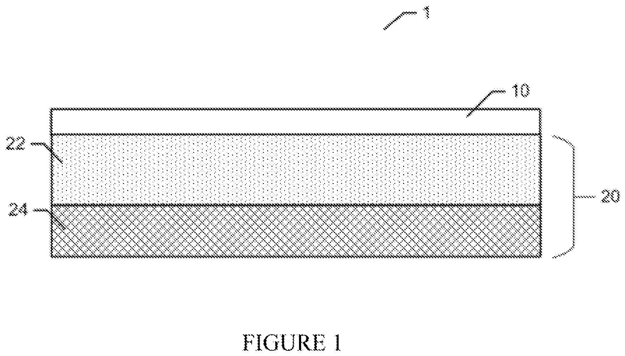

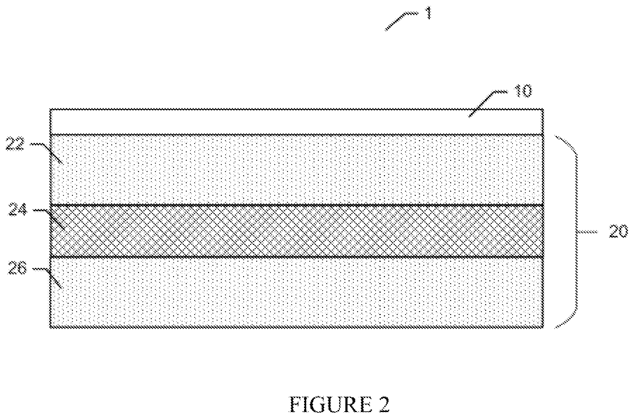

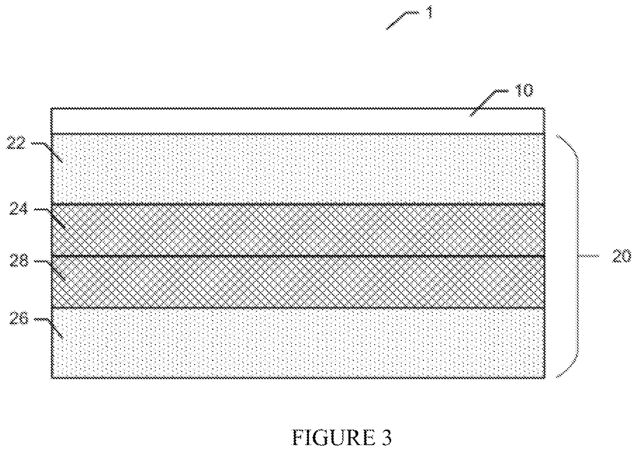

Figure 1, for instance, illustrates an absorbent composite according to certain embodiments of the invention. The absorbent composite 1 illustrated in Figure 1 includes a nonwoven top layer 10 bonded to an absorbent core 20. The absorbent core includes a first physically entangled nonwoven layer 22 and a first bonded carded nonwoven layer 24. Figure 2 illustrates an absorbent composite 1 including an absorbent core 20 having two physically entangled nonwoven layers 22, 24 in accordance with certain embodiments of the invention. The absorbent core illustrated by Figure 2 includes the first bonded carded web 24 located between the first physically entangled nonwoven layer 22 and a second physically entangled nonwoven layer 26. Figure 3 illustrates yet another absorbent composite according to certain embodiments of the invention, in which the absorbent composite includes two bonded carded nonwoven layers 24, 28 located between two physically entangled nonwoven layers 22, 26. In this regard, the first bonded carded nonwoven layer 24 and a second bonded carded nonwoven layer 28 are adjacent and positioned between the first physically entangled nonwoven layer 22 and the second physically entangled nonwoven layer 28. In accordance with certain embodiments of the invention, the first carded bonded nonwoven layer and the second carded bonded nonwoven layer may be bonded together by a variety of bonds (e.g., thermal bonds, ultrasonic bonds, adhesive bonds, etc.). Although Figure 1-3 illustrate particular embodiments with a particular number or respective layers, the absorbent composite may include from 1 to about 5 physically entangled nonwoven layers and/or from 1 to about 5 bonded carded nonwoven layers.

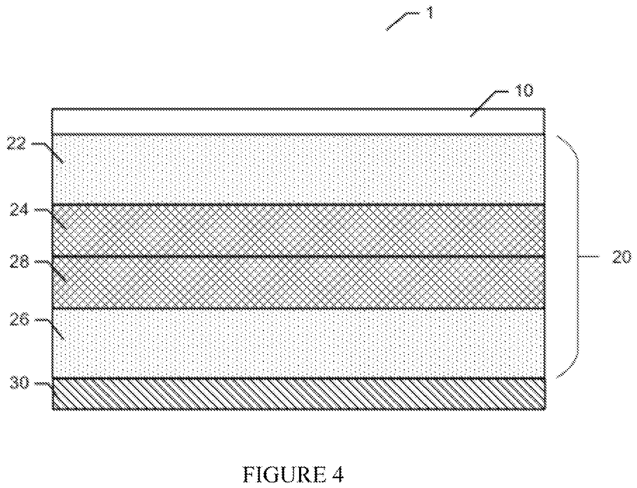

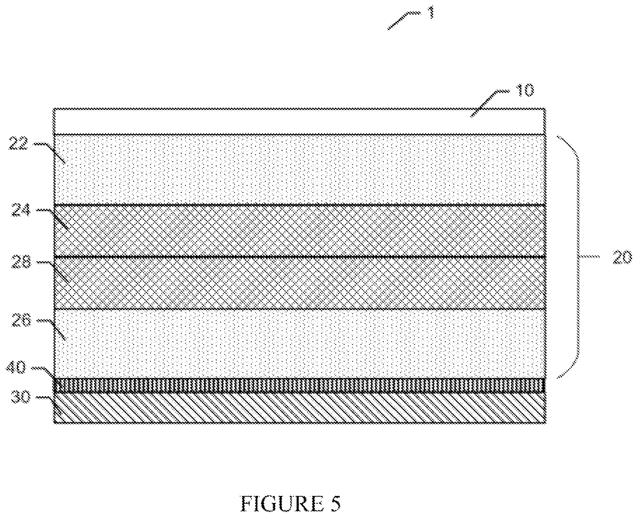

Figure 4 illustrates an absorbent composite 1 including the basic structure of the absorbent composite illustrated in Figure 3, but includes a film layer 30 directly attached to the absorbent core 20. In this regard, the film layer 30 may be directly bonded to the second physically entangled nonwoven layer 26 via thermal or ultrasonic bonding. In accordance with certain embodiments of the invention, the film layer 30 may be extrusion coated directly onto the lowermost layer of the absorbent core. Figure 5 illustrates an absorbent composite 1 including the basic structure of the absorbent composite illustrated in Figure 3, but includes a film layer 30 adhesively bonded to the absorbent core by and adhesive layer 40 in accordance with certain embodiments of the invention.

In accordance with certain embodiments of the invention, the nonwoven top layer may comprise a spunmelt nonwoven, such as a spunbond nonwoven, meltblown nonwoven, or a combination thereof. For example, the nonwoven top layer may comprise one or more meltblown layer and one or more spunbond layers. In accordance with certain embodiments of the invention, the nonwoven top layer may comprise a S1ₐ-M_{b}-S2_{c} structure; wherein 'S1' is a first spunbond material, 'M' is a meltblown material, 'S2' is a second spunbond material, and 'a', 'b', and 'c' indicate the number of respective layers and each may independently be selected from a value of at least 1, such as 1, 2, 3, 4, or 5. In accordance with certain embodiments of the invention, the nonwoven top layer may comprise, additionally or alternatively to a spunmelt nonwoven, a carded web comprising staple fibers, such as a point bonded carded web.

The nonwoven top layer, in accordance with certain embodiments of the invention, may comprise at least about 30% by weight of hydrophilic fibers, such as at least about 40%, at least about 50%, at least about 60%, at least about 70%, at least about 80%, at least about 90%, or about 100% by weight of hydrophilic fibers. In accordance with certain embodiments of the invention, for instance, the nonwoven top layer may comprise at most about any of the following: 100%, 95%, 90%, 80%, 70%, 60%, 50%, and 40% by weight of hydrophilic fibers and/or at least about any of the following: 20%, 25%, 30%, 35%, 40%, 50%, 60%, 70%, 80%, and 90% by weight of hydrophilic fibers.

In accordance with certain embodiments of the invention, the nonwoven top layer may comprise a generally "open" structure that facilitates the penetration of fluid through the nonwoven top layer. The "openness" of the nonwoven top layer may be evaluated by air permeability of the nonwoven top layer, in which a lower air permeability may be indicative a more closed structure and a higher air permeability may be indicative of a relatively more "open" structure. In accordance with certain embodiments of the invention, for example, the nonwoven top layer may include an air permeability of at least about 100 cubic-feet-per-minute (CFM) as determined by IST 70.1, such as at least about 150 CFM, at least about 200 CFM, at least about 250 CFM, at least about 300 CFM, at least about 350 CFM, at least about 400 CFM, or at least about 500 CFM as determined by IST 70.1. In accordance with certain embodiments of the invention, for instance, the nonwoven top layer may include an air permeability of at most about any of the following: 800, 700, 600, 500, 400, 350, 300, and 250 CFM as determined by IST 70.1 and/or at least about any of the following: 40, 50, 75, 100, 125, 150, 175, 200, 225, and 250 CFM as determined by IST 70.1.

The nonwoven top layer, in accordance with certain embodiments of the invention, may have a thickness in the z-direction, which is perpendicular to a cross-direction and a machine direction) from about 0.03 mm to about 0.3 mm, such as at least about any of the following: 0.03, 0.05, 0.08, 0.1, 0.15, 0.18, and 0.2 mm, and/or at most about any of the following: 0.3, 0.28, 0.25, 0.22, and 0.2 mm.

The nonwoven top layer, in accordance with certain embodiments of the invention, may comprise a pre-bonded nonwoven including a bonding area. For instance, the nonwoven top layer may comprise a bonding area prior to formation of the absorbent composite comprising from about 1-30% of the surface of the nonwoven top layer, such as from about 1-25%, from about 3-20%, or from about 5-15%. In accordance with certain embodiments of the invention, for instance, the nonwoven top layer may comprise a bonding area prior to formation of the absorbent composite comprising at most about any of the following: 30%, 25%, 20%, 15%, 10%, and 5% of the surface of the nonwoven top layer and/or at least about any of the following: 1%, 3%, 5%. 8%, 10%, 12%, 15%, 18%, and 20% of the surface of the nonwoven top layer. In this regard, the nonwoven top layer may include a bonding pattern prior to formation of the absorbent composite comprising, for example, thermally-formed point bonds, ultrasonic bonds, mechanical bonds, or any combination thereof.

In accordance with certain embodiments of the invention, the nonwoven top layer comprises a desirable resistance to abrasion, which mitigates the formation of lint when in use. Absorbent composites including the nonwoven top layer may be tested for abrasion resistance, in which the nonwoven top layer is directly subjected to the test (i.e., IST 20.5) and comprises a top-layer-Martindale Abrasion value of from about 2 mg to about 15 mg as determined by IST 20.5, such at most about any of the following: 15, 14, 13, 12, 11, 10, 9, 8 and 7 mg as determined by IST 20.5 and/or at least about any of the following: 2, 3, 4, 5, 6, and 7 mg as determined by IST 20.5. In accordance with certain embodiments of the invention, the absorbent composites including the nonwoven top layer may be tested in which the nonwoven top layer is directly tested and comprises a top-layer-abrasion cycle value of at most about any of the following: 7000, 6000, 5750, 5500, 5250, and 5000 cycles as determined by ASTM D4966 and/or at least about any of the following: 3000, 3500, 4000, 4500, and 5000 as determined by ASTM D4966.

The nonwoven top layer, in accordance with certain embodiments of the invention, may comprises a basis weight from about 5-60 grams-per-square-meter (gsm), such as from about 15-50 gsm, 20-50 gsm, 25-45 gsm, 25-40 gsm, or from about 25-35 gsm. For instance, the nonwoven top layer may comprise a basis weight of at least about any of the following: 5, 6, 8, 10, 12, 15, 20, 25, and 30 gsm, and/or at most about any of the following: 60, 50, 40, and 30 gsm.

As noted above, the nonwoven top layer comprises a plurality of hydrophilic fibers. Such fibers may be rendered hydrophilic via topical application or treatment with a hydrophilic additive and/or a hydrophilic additive may be incorporated into the polymer melt used to form the plurality of hydrophilic fibers used, at least in part, for formation of the nonwoven top layer. In this regard, the plurality of hydrophilic fibers of the nonwoven top layer may comprise monocomponent fibers, multicomponent fibers, or a combination thereof. In accordance with certain embodiments of the invention, the hydrophilic fibers of the nonwoven top layer include monocomponent fibers comprising one or more synthetic polymers, such as a polyolefins, polyesters, polyamides, polylactic acid, polyglycolic acid, or any combination thereof. Examples of suitable polyolefins includes, for example, a polypropylene, a polyethylene, copolymers thereof, or blends thereof. As noted above, these fibers may be rendered hydrophilic to facilitate intake of liquids, such as water and blood. In accordance with certain embodiments of the invention, the hydrophilic fibers of the nonwoven top layer include multicomponent fibers, such as bicomponent fibers including a sheath-and-core configuration and/or a side-by-side configuration. In accordance with certain embodiments of the invention, the nonwoven top layer comprises hydrophilic bicomponent fibers, such as bicomponent fibers including a sheath comprising a polyolefin (e.g., a polyethylene) and a core comprising at least one of a polyolefin (e.g., a polypropylene) or a polyester.

In accordance with certain embodiments of the invention, the absorbent core may include only a single physically entangled nonwoven layer and a single bonded carded nonwoven layer as illustrated by Figure 1. In such embodiments, the single physically entangled nonwoven layer may be directly or indirectly located between the nonwoven top layer and the single bonded carded nonwoven layer.

In accordance with certain embodiments of the invention, the absorbent core may comprise at least two physically entangled nonwoven layers and at least one bonded carded nonwoven layer and/or at least one thermal bonded high-loft nonwoven layer. For example, the absorbent core may comprise a first physically entangled nonwoven layer, a second physically entangled nonwoven layer, and a first bonded carded nonwoven layer (or a first thermal bonded high-loft nonwoven layer) as illustrated by Figure 2. The first bonded carded nonwoven layer (or a first thermal bonded high-loft nonwoven layer) may be directly or indirectly located between the first physically entangled nonwoven layer and the second physically entangled nonwoven layer. In accordance with certain embodiments of the invention, the first bonded carded nonwoven layer (or a first thermal bonded high-loft nonwoven layer) may be adjacent and/or in contact with both the first and second physically entangled nonwoven layers.

In accordance with certain embodiments of the invention, the absorbent core may comprise at least two physically entangled nonwoven layers and at least two bonded carded nonwoven layers and/or at least two thermal bonded high-loft nonwoven layers. For example, the absorbent core may comprise a first physically entangled nonwoven layer, a second physically entangled nonwoven layer, a first bonded carded nonwoven layer (or a first thermal bonded high-loft nonwoven layer), and a second bonded carded nonwoven layer (or a second thermal bonded high-loft nonwoven layer) as illustrated by Figure 3. In this regard, the first bonded carded web or the first thermal bonded high-loft nonwoven layer may be directly or indirectly located between the first physically entangled nonwoven layer and the second bonded carded nonwoven layer. In accordance with certain embodiments of the invention, the first and second bonded carded nonwoven layers (or the first and second thermal bonded high-loft nonwoven layers) may be adjacent and/or in contact with each other while the first bonded carded web (or the first thermal bonded high-loft nonwoven layer) may be adjacent and/or in contact with the first physically entangled nonwoven layer. In accordance with certain embodiments of the invention, the first bonded carded nonwoven layer may be bonded to the second bonded nonwoven layer (or the first thermal bonded high-loft nonwoven layer may be bonded to the second thermal bonded high-loft nonwoven layer), such as by one or more thermal bonds, ultrasonic bonds, mechanical bonds, adhesive bonds, or any combination thereof.

In accordance with certain embodiments of the invention, the first physically entangled nonwoven layer has a basis weight from about 20 to 150 gsm, such as at least about any of the following: 20, 25, 30, 35, 40, 45, 50, 55, 60, 65, 70, and 75 gsm, and/or at most about any of the following: 150, 140, 130, 120, 115, 110, 105, 100, 95, 90, 85, and 75 gsm. Additionally or alternatively, the second physically entangled nonwoven layer has a basis weight from about 20 to 100 gsm, such as at least about any of the following: 20, 25, 30, 35, 40, 45, 50, 55, and 60 gsm, and/or at most about any of the following: 100, 90, 80, 75, 70, 65, and 60 gsm.

In accordance with certain embodiments of the invention, the first physically entangled nonwoven layer, the second physically entangled nonwoven layer, or both independently (from each other) may include from about 10% to about 90% by weight of synthetic polymeric fibers, such as at least about any of the following: 10, 15, 20, 25, 30, 35, 40, 45, and 50% by weight of synthetic polymeric fibers, and/or at most about any of the following: 90, 80, 70, 60, and 50% by weight of synthetic polymeric fibers. The synthetic polymeric fibers may comprise a synthetic polymer comprising polyolefin, a polyester, a polyamide, or any combination thereof. In accordance with certain embodiments, the synthetic polymer is a polyester. Additionally or alternatively, the synthetic polymeric fibers comprise from about 20%) to about 100% by weight of hydrophilic fibers, such as at least about any of the following: 20, 30, 40, and 50% by weight of hydrophilic fibers, and/or at most about any of the following: 100, 90, 80, 70, 60, and 50% by weight of hydrophilic fibers. As noted above, the hydrophilic fibers of the first physically entangled nonwoven layer, the second physically entangled nonwoven layer, or both may independently comprise a hydrophilic coating thereon. Additionally or alternatively, the hydrophilic fibers of the first physically entangled nonwoven layer, the second physically entangled nonwoven layer, or both may independently comprise a hydrophilic additive dispersed throughout the synthetic polymeric material.

In accordance with certain embodiments of the invention, the first physically entangled nonwoven layer, the second physically entangled nonwoven layer, or both may independently include from about 10% to about 90% by weight of cellulosic fibers, such as at least about any of the following: 10, 15, 20, 25, 30, 35, 40, 45, and 50% by weight of cellulosic polymeric fibers, and/or at most about any of the following: 90, 80, 70, 60, and 50% by weight of cellulosic fibers. The cellulosic fibers may comprise, for example, cellulose acetate, regenerated cellulose (e.g., viscose, rayon), saponified acetate, or any combination thereof.

In accordance with certain embodiments of the invention, the first physically entangled nonwoven layer, the second physically entangled nonwoven layer, or both may independently include a plurality of apertures extending through an entire thickness in the z-direction, which is perpendicular to an x-y plane. The plurality of apertures may comprises a first plurality of apertures of the first physically entangled nonwoven layer may define a first open area, in which the first open area comprises from about 1% to about 30%, such as at least about any of the following: 1, 2, 3, 5, 6, 8, 10, 12, 14, and 15%, and/or at most about any of the following: 30, 28, 26, 25, 24, 22, 20, 18, 16, and 15%. Additionally or alternatively, the plurality of apertures may comprises a second plurality of apertures of the first physically entangled nonwoven layer may define a second open area, in which the second open area comprises from about 1% to about 30%, such as at least about any of the following: 1, 2, 3, 5, 6, 8, 10, 12, 14, and 15%, and/or at most about any of the following: 30, 28, 26, 25, 24, 22, 20, 18, 16, and 15%.

The plurality of apertures, in accordance with certain embodiments of the invention may have an average diameter from about 0.1 mm to about 3 mm, such as at least about any of the following: 0.1, 0.2, 0.4, .05. 0.6, 0.8, and 1 mm, and/or at most about any of the following: 3, 2.5, 2, 1.8, 1.6, 1.5, 1.4, 1.2, and 1 mm. In accordance with certain embodiments of the invention, the plurality of apertures may not be circular. For example, the plurality of apertures may be provided in a variety of shapes, such as squares, rectangles, ellipses, etc. In this regard, the average diameter may be considered to be the longest dimension across the aperture. By way of example only, a rectangular aperture having a width of 0.5 mm and a length of 1 mm would have a diameter, as used herein, of 1.12 mm. In accordance with certain embodiments of the invention, the second physically entangled nonwoven layer (i.e., furthest from the top nonwoven layer) may be a smaller open area and/or smaller apertures than the first physically entangled nonwoven layer (i.e., closest to the top nonwoven layer).

In accordance with certain embodiments of the invention, the absorbent core may include a plurality of bonded carded nonwoven layers, such as a first bonded carded nonwoven layer and a second bonded nonwoven carded layer. In this regard, each bonded carded nonwoven layer may independently have a basis weight from about 20 to 60 gsm, such as at least about any of the following: 20, 25, 30, 35, and 40, gsm, and/or at most about any of the following: 60, 55, 50, 45, and 40 gsm. Additionally or alternatively, the first bonded carded nonwoven layer, the second bonded carded nonwoven layer, or both may independently comprise a plurality of synthetic staple fibers comprising a synthetic polymeric material, such as a polyolefin, a polyester, a polyamide, or any combination thereof. In accordance with certain embodiments of the invention, the synthetic polymer is a polyester.

The plurality of synthetic staple fibers, for example, may comprise from about 20% to about 100% by weight of hydrophilic staple fibers, such as at least about any of the following: 20, 30, 40, and 50% by weight of hydrophilic staple fibers, and/or at most about any of the following: 100, 90, 80, 70, 60, and 50% by weight of hydrophilic staple fibers. In accordance with certain embodiments of the invention, the hydrophilic staple fibers of the first bonded carded nonwoven layer, the second bonded carded nonwoven layer, or both may independently comprise a hydrophilic coating thereon. Additionally or alternatively, the hydrophilic staple fibers of the first bonded carded nonwoven layer, the second bonded carded nonwoven layer, or both may independently comprise a hydrophilic additive dispersed throughout the synthetic polymeric material.

In accordance with certain embodiments of the invention, the first bonded carded nonwoven layer, the second bonded carded nonwoven layer, or both may independently comprise crimped staple fibers. The first bonded carded nonwoven layer, the second bonded carded nonwoven layer, or both may independently have a void volume greater than 9 cc/g, such as greater than about 12 cc/g, greater than about 14 cc/g, greater than about 15 cc/g, greater than about 16 cc/g, greater than about 17 cc/g, greater than about 18 cc/g, greater than about 19 cc/g, greater than about 20 cc/g, greater than about 25 cc/g, or greater than about 30 cc/g. Additionally or alternatively, the first bonded carded nonwoven layer, the second bonded carded nonwoven layer, or both may independently have a thickness from about 0.02 cm to about 0.15 cm, such as at least about any of the following: 0.02, 0.04, 0.05, 0.06, 0.08, and 0.1 cm, and/or at most about any of the following: 0.15, 0.1475, 0.145, 0.14, 0.135, 0.13, 0.125, 0.12, 0.115, 0.11, 0.105, and 0.1 cm. By way of example only, using 40 gsm bonded carded nonwoven layer with 15 denier 100% PET fiber at Density 1.38 g/cm³ may have a thickness of around 0.1375 cm to about 0.1475 cm with a void volume (VO) from about 33 cc/g to about 36 cc/g. In accordance with certain embodiments of the invention, the first bonded carded nonwoven layer, the second bonded carded nonwoven layer, or both may independently comprise a high loft nonwoven layer. By way of example only, a high loft nonwoven layer made of a first polypropylene and a second polypropylene having a basis weight of 18 gsm with 1.5 denier fibers at density about 0.905 g/cm³ at a thickness of 0.0431 cm may have a void volume (VO) 22.84 cc/g while such a high loft nonwoven layer at a thickness of 0.0570 cm may have a void volume (VO) of about 30.56 cc/g.

In accordance with certain embodiments of the invention and as noted above, each of the bonded carded nonwoven layers may be replaced with thermal bonded high-loft nonwoven layers. In each of the figures illustrating one or more bonded carded nonwoven layers, for instance, a corresponding number of thermal bonded high-loft nonwoven layers may be substituted in place of the bonded carded nonwoven layers. In accordance with certain embodiments of the invention, for example, the absorbent core may comprise, for example, a first thermal bonded high-loft nonwoven layer and a second thermal bonded high-loft nonwoven layer, in which the first thermal bonded high-loft nonwoven layer, the second thermal bonded high-loft nonwoven layer, or both may independently comprise a plurality of synthetic staple fibers and/or continuous fibers having an average denier from about 1 to about 35 denier, such as at least about any of the following: 1, 2, 3, 5, 6, 8, 10, 15, and 20 denier, and/or at most about any of the following: 35, 30, 25, 20, 15, 14, 12, and 10 denier.

In accordance with certain embodiments of the invention, the first thermal bonded high-loft nonwoven layer, the second thermal bonded high-loft nonwoven layer, or both may independently have a basis weight from about 10 to 40 gsm, such as at least about any of the following: 10, 12, 15, 18, 20, 22, 24, and 25 gsm, and/or at most about any of the following: 40, 38, 35, 32, 30, 28, 26, and 25 gsm. Additionally or alternatively, the first thermal bonded high-loft nonwoven layer, the second thermal bonded high-loft nonwoven layer, or both may independently comprise a plurality of synthetic staple fibers and/or synthetic continuous fibers comprising a synthetic polymeric material, such as a polyolefin, a polyester, a polyamide, or any combination thereof. In accordance with certain embodiments of the invention, the synthetic polymeric material may comprise a blend of a first polyolefin and a second polyolefin that is different from the first polyolefin. For example, the first polyolefin may comprise a first polypropylene and the second polyolefin may comprise a polypropylene-based copolymer, such as a polypropylene-polyethylene copolymer. In accordance with certain embodiment of the invention, the polypropylene-polyethylene copolymer may comprise from about 1 to about 50 wt.% of polyethylene, such as at least about any of the following: 1, 3, 5, 8, 10, 12, 15, 18, 20, 22, and 25 wt.% of polyethylene, and/or at most about any of the following: 50, 45, 40, 35, 30, 28, and 25 wt.% of polyethylene. In accordance with certain embodiment of the invention, the total amount of ethylene content in a thermal bonded hi-loft nonwoven layer may comprise from about 1 wt.% to about 40 wt.%, such as at least about any of the following: 1, 2, 3, 4, 5, 6, 8, 10, 12, 15, 18, and 20 wt.% of ethylene content, and/or at most about any of the following: 40, 35, 30, 25, and 20 wt.% of ethylene content.

The plurality of plurality of synthetic staple fibers and/or synthetic continuous fibers, for example, may comprise from about 20% to about 100% by weight of hydrophilic staple fibers and/or hydrophilic continuous fibers, such as at least about any of the following: 20, 30, 40, and 50% by weight of hydrophilic staple fibers, and/or at most about any of the following: 100, 90, 80, 70, 60, and 50% by weight of hydrophilic staple fibers. In accordance with certain embodiments of the invention, the hydrophilic staple fibers and/or hydrophilic continuous fibers the first thermal bonded high-loft nonwoven layer, the second thermal bonded high-loft nonwoven layer, or both may independently comprise a hydrophilic coating thereon. Additionally or alternatively, the hydrophilic staple fibers and/or hydrophilic continuous fibers of the first thermal bonded high-loft nonwoven layer, the second thermal bonded high-loft nonwoven layer, or both may independently comprise a hydrophilic additive dispersed throughout the synthetic polymeric material.

In accordance with certain embodiments of the invention, the synthetic staple fibers and/or synthetic continuous fibers forming one or more (e.g., all) of the thermal bonded hi-loft nonwoven layer(s) (e.g., the first thermal bonded high-loft nonwoven layer, the second thermal bonded high-loft nonwoven layer, or both) may independently from each other comprise form 0 to 100 % by number of crimped fibers, such as at least about any of the following: 0, 5, 10, 15, 20, 25, 30, 35, 40, 45, and 50 % by number of crimped fibers, and/or at most about any of the following: 100, 95, 90, 85, 80, 75, 70, 65, 60, 55, and 50 % by number of crimped fibers.

In accordance with certain embodiments of the invention, each of the thermal bonded hi-loft nonwoven layer(s) (e.g., the first thermal bonded high-loft nonwoven layer, the second thermal bonded high-loft nonwoven layer, or both) may have a thickness in the z-direction, which is perpendicular to a cross-direction and a machine direction) from about 0.2 mm to about 1 mm, such as at least about any of the following: 0.2, 0.3, 0.4, and 0.5 mm, and/or at most about any of the following: 1, 0.9. 0.8, 0.7, 0.6, and 0.5 mm. Additionally or alternatively, each of the thermal bonded hi-loft nonwoven layer(s) (e.g., the first thermal bonded high-loft nonwoven layer, the second thermal bonded high-loft nonwoven layer, or both) may have an air permeability from about 300 to about 800 CFM, such as at least about any of the following: 300, 325, 350, 375, 400, 425, 450, 475, 500, and 525 CFM, and/or at most about any of the following: 800, 775, 750, 725, 700, 675, 650, 625, 600, 575, 550, and 525 CFM.

In accordance with certain embodiments of the invention, each of the thermal bonded hi-loft nonwoven layer(s) (e.g., the first thermal bonded high-loft nonwoven layer, the second thermal bonded high-loft nonwoven layer, or both) may have a void volume from about 4 to about 15 g/cc, such as at least about any of the following: 4, 5, 6, 7, 8, 9, and 10 cc/g, and/or at most about any of the following: 15, 14, 13, 12, 11, and 10 cc/g.

In accordance with certain embodiments of the invention, the absorbent core may be devoid of natural cellulosic materials and/or synthetic cellulosic materials. In accordance with certain alternative embodiments of the invention, the absorbent core may comprise natural cellulosic materials and/or synthetic cellulosic materials. The absorbent core, in accordance with certain embodiments of the invention, may be devoid of pulp, meltblown fibers, or both. Additionally or alternatively, the absorbent core may be devoid of through-air-bonded nonwoven layers.

In accordance with certain embodiments of the invention, the absorbent composite includes a film layer bonded to the absorbent core, in which the absorbent core is located directly or indirectly between the film layer and the nonwoven top layer. The film layer, for example, may comprise a water impermeable film. Additionally or alternatively, the film layer may comprise at least one polyolefin, such as polyethylene.

In accordance with certain embodiments of the invention, the film layer may be adhesively bonded to the absorbent core via an adhesive layer. In this regard, the adhesive layer may be located directly between the film layer and the absorbent core (e.g., the layer of the absorbent core that is furthest away from the top nonwoven layer). Alternatively, the film layer may be melt extruded directly onto the absorbent core (e.g., the layer of the absorbent core that is furthest away from the top nonwoven layer).

The film layer, in accordance with certain embodiments of the invention, may have a basis weight from about 5 to about 50 gsm, such as at least about any of the following: 5, 8, 10, 12, 15, 18, 20, 22, 25, 28, and 30 gsm, and/or at most about any of the following: 50, 45, 40, 35, and 30 gsm. Additionally or alternatively, the adhesive layer (when present) may have a basis weight from about 1 to about 10 gsm, such as at least about any of the following: 1, 2, 3, 4, and 5 gsm, and/or at most about any of the following: 10, 9, 8, 7, 6, and 5 gsm.

In accordance with certain embodiments of the invention, the nonwoven top layer and the absorbent core are directly or indirectly bonded together via a composite-bonding pattern formed from thermally-formed point bonds, ultrasonic bonds, mechanical bonds, adhesive (e.g., binder or glue) bonds, or any combination thereof. The composite-bonding pattern, in accordance with certain embodiments of the invention, may comprise a plurality of ultrasonic point bonds.

The composite-bonding pattern, in accordance with certain embodiments of the invention, may define a composite-bonding area of no more than 30% of a surface of the absorbent composite, such as no more than about 25%, no more than about 20%, no more than about 15%, no more than about 10%, no more than about 5%, or no more than about 3% of a surface of the absorbent composite. In accordance with certain embodiments of the invention, for instance, the composite-bonding area may comprise at most about any of the following: 30%, 25%, 20%, 15%, 10%, 5%, and 3% of the surface of the absorbent composite and/or at least about any of the following: 0.5%, 1%, 2%, 3%, 5%, 8%, 10%, 12%, and 15% of the surface of the absorbent composite.

As noted above, certain embodiments of the invention comprise one or more desirable properties for an absorbent composite suitable for a variety of uses. For example, the absorbent composite comprises a composite-run-off value of less than 15% as determined by ISO 9073-11, such as less than 12%, less than 10%; less than 8%, less than 6%, less than 4%, less than 3%, or less than 1% as determined by ISO 9073-11. In accordance with certain embodiments of the invention, for instance, absorbent composite may comprise a composite-run-off value from about 0.25 to about 15% as determined by ISO 9073-11, such as at least about any of the following: 0.25, 0.5, 0.75, 1, 2, 3, 4, 5, 6, 7, 8, 9, and 10%, and/or at most about 15, 14, 13, 12, 11, and 10% as determined by ISO 9073-11.

The absorbent composite, in accordance with certain embodiments of the invention, may comprise a composite-absorption capacity of at least 600% as determined by ISO 9073, such as at least 650%, at least 700%, at least 725%, at least 750%, at least 775%, at least 800%, at least 825%, at least 850%, at least 875%, at least 900%, at least 950%, at least 1000%, or at least about 1500% as determined by ISO 9073. In accordance with certain embodiments of the invention, for instance, absorbent composite may comprise a composite-absorption capacity at most about any of the following: 1500%, 1400%, 1300%, 1200%, 1100%, 1000%, 950%, 900%, 875%, 850%, 825%, and 800% as determined by ISO 9073 and/or at least about any of the following: 500%, 550%, 600%, 700%, and 725% as determined by ISO 9073.

The absorbent composite comprises a composite-rate of absorption of less than 8 seconds for a 5 ml liquid sample as determined by D824-94, such as less than 7 second, less than 6 second, less than 5 second, or less than 4 second as determined by D824-94. In accordance with certain embodiments of the invention, for instance, absorbent composite may comprise a composite-rate of absorption at most about any of the following: 8, 7, 6, and 5 seconds as determined by D824-94 and/or at least about any of the following: 3, 4, 5, and 6 second as determined by D824-94.

The absorbent composite, in accordance with certain embodiments of the invention, may comprise a composite-void volume of at least 7 g/cc, such as at least 8 cc/g, or at least 9 cc/g. In accordance with certain embodiments of the invention, for instance, absorbent composite may comprise a composite-void volume at most about any of the following: 12, 10, 9, and 8 cc/g and/or at least about any of the following: 1, 2, 3, 4, 5, 6, and 7 cc/g.

The absorbent composite, in accordance with certain embodiments of the invention, may comprise a composite-Martindale Abrasion value of less than about 2 mg as determined by IST 20.5, such as less than about 1.75 mg, less than about 1.5 mg, less than about 1.25 mg, less than about 1.0 mg, less than about 0.75 mg, or less than about 0.50 mg as determined by IST 20.5. In accordance with certain embodiments of the invention, for instance, the absorbent composite may include a composite-Martindale Abrasion value of at most about any of the following: 3, 2.5, 2, 1.5, 1, 0.75, and 0.5 mg as determined by IST 20.5 and/or at least about any of the following: 0.25, 0.50, 0.75, 1.0, and 1.25 mg as determined by IST 20.5. In accordance with certain embodiments of the invention, the absorbent composite may comprise a composite-abrasion cycle value of at most about any of the following: 7000, 6000, 5750, 5500, 5250, and 5000 cycles as determined by ASTM D4966 and/or at least about any of the following: 3000, 3500, 4000, 4500, and 5000 as determined by ASTM D4966.

The absorbent composite, in accordance with certain embodiments of the invention, may comprise a composite-water-absorption ratio between the weight of water absorbed by the absorbent composite to the dry weight of the composite from 6 : 1 to 15: 1 as determined by ISO 9073, such as from 6 : 1 to 12: 1, from about 7 : 1 to 10 : 1, from about 7 : 1 to 9 : 1, from about 7 : 1 to 8.5 : 1, or from 7.5 : 1 to 8.5 : 1.

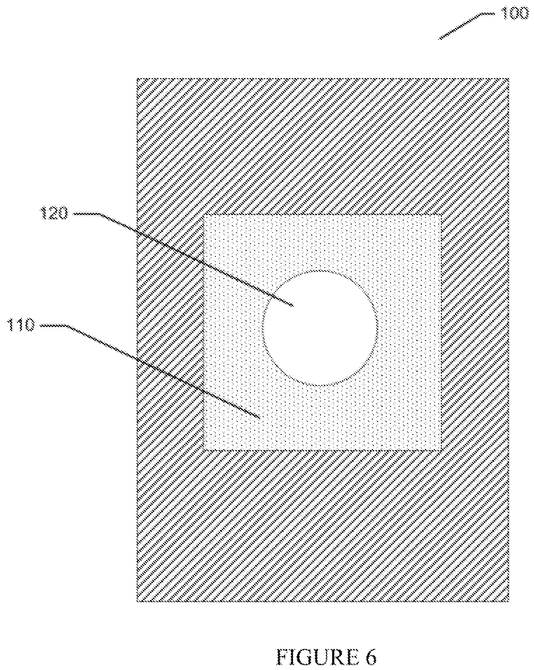

As noted above, absorbent composites in accordance with certain embodiments of the invention may comprise a combination of properties suitable for a variety of applications in which rapid liquid absorption, absorption capacity, and/or limited run-off while maintaining desirable resistance to abrasion. In accordance with certain embodiments of the invention, for example, the absorbent composite (as disclosed herein) may be provided in the form of a surgical drape, a portion of a surgical drape, a tray liner, or an under-patient absorbent pad. In accordance with certain embodiments of the invention, the absorbent composite comprises a fenestration material surrounding a fenestration through which a surgical procedure can be performed. For example, Figure 6 illustrates a surgical drape 100 including an absorbent composite 110 disposed around a fenestration 120 through with a medical (e.g., surgical) procedure may be performed.

In another aspect, the invention not claimed provides a material suitable for use, for example alone or when incorporated into an article of manufacture, in the healthcare industry for the prevention and/or treatment of skin breakdown, which can undesirably lead to complications such as decubitus ulcers. A patient, for example, may experience skin breakdown at or during several points throughout the care of a patient in a hospital, a nursing home, or a homecare setting. In accordance with certain embodiments of the invention, for instance, an absorbent composite either alone or as part of an article of manufacture (e.g., adult diaper, bedding sheet, gown, liners, underpads, surgical underlays, etc.) that may be placed in contact with the skin of a patient. Certain embodiments not according to the invention also provide methods of preventing skin deterioration (e.g., decubitus ulcers) of an individual susceptible to development of skin deterioration (e.g., decubitus ulcers). Individuals to susceptible to development of skin deterioration may include any patient that may spend a considerable amount of time one or a few positions (e.g., a patient that is mostly or wholly confined to a bed) over the course of multiple days. In this regard, absorbent composites in accordance with certain embodiments of the invention may provide a micro-climate environment at or adjacent the skin of an individual having one or more of a desirable air permeability, a low coefficient of friction, and/or highly absorbent for proper humidity levels. In another aspect, certain embodiments not according to the invention also provide methods of treating individuals already suffering or showing signs of skin deterioration (e.g., decubitus ulcers). In this regard, the absorbent composites in accordance with certain embodiments of the invention may provide a micro-climate environment (as noted above) at or adjacent the skin of the individual already suffering or showing signs of skin deterioration (e.g., decubitus ulcers) such that the rate or severity of the skin deterioration may be positively impacted (e.g., rate of deterioration may be slowed, stopped, and/or reversed.

In another aspect, the invention provides a method of making an absorbent composite including the following steps: (i) providing a nonwoven top layer comprising hydrophilic fibers;
(ii) providing an absorbent core, in which the absorbent core comprises a plurality of layers including at least one physically entangled nonwoven layer and at least one bonded carded nonwoven layer; and (iii) directly or indirectly attaching the nonwoven top layer and the absorbent core to provide the absorbent composite as disclosed described herein. In accordance with certain embodiments of the invention, the method may further comprise topically treating the nonwoven top layer, the absorbent core, or both with a hydrophilic additive. Methods, in accordance with certain embodiments of the invention, may further comprise attaching a film directly or indirectly to the absorbent core, wherein the absorbent core layer is directly or indirectly sandwiched between the nonwoven top layer and the film layer. In accordance with certain embodiments of the invention, the step of attaching the film layer to the absorbent core may comprise adhesively laminating the film to the absorbent core layer, for example via a continuous or discontinuous layer of adhesive (e.g., a pressure-sensitive adhesive). In accordance with certain other embodiments of the invention, the step of attaching the film to the absorbent core layer may comprise extrusion coating the film onto the absorbent core (e.g., the lowest most layer of the absorbent core).

Additionally or alternatively, the method may further comprise forming a first polymer melt including a hydrophilic additive and forming the hydrophilic fibers of the nonwoven top layer and/or forming a second polymer melt including a hydrophilic additive and forming absorbent core fibers (e.g., included in the at least one physically entangled nonwoven layer and/or the at least one bonded carded nonwoven layer).

In accordance with certain embodiments of the invention, the method may comprise bonding (e.g., thermal bonds, ultrasonic bonds, adhesive bonds, etc.) a first physically entangled nonwoven layer to a first bonded carded nonwoven layer, and bonding (e.g., thermal bonds, ultrasonic bonds, adhesive bonds, etc.) a second physically entangled nonwoven layer to a second bonded carded nonwoven layer to provide two intermediate core layers. The two intermediate layers may then be bonded (e.g., thermal bonds, ultrasonic bonds, adhesive bonds, etc.) together to form the absorbent core. The absorbent core may then be bonded (e.g., thermal bonds, ultrasonic bonds, adhesive bonds, etc.) to the top nonwoven layer. A film layer may optionally be attached to a free or exposed side of the absorbent core in any manner described and disclosed herein.

### Examples

The present disclosure is further illustrated by the following examples, which in no way should be construed as being limiting. That is, the specific features described in the following examples are merely illustrative and not limiting.

### Test Methods

Absorption capacity has been measured as per ISO 9073 test method and expressed in percentage (%) that represent the weight of liquid absorbed relative to the weight of the dry sample.

Water Absorbed Per Square Inch is determined by test method ISO 9073, in which the weight of water absorbed in grams is divided by the area (expressed in a square inches) of the sample tested.

Water Absorbed Per Sample Weight is determined by dividing the weight of the water absorbed by the sample as determined by ISO 9073 by the weight of the sample prior to absorbing any liquid.

Rate of Absorption is the rate of absorption as determined per ASTM method D824-94 where the liquid used is deionized water. The composite is used as the sample being tested and the volume of dispensed liquid is 1 ml for a fist set of tests and 5 ml for a second set of tests.

### Example Set (1)

A 60 gsm spunlace nonwoven layer was formed and evaluated for a variety of physical properties. The spunlace nonwoven layer included 70% by weight of viscose rayon fibers and 30%, by weight of polyester fibers, which were rendered hydrophilic. A 40 gsm bonded carded nonwoven layer was also prepared and evaluated for the same properties as the 60 gsm spunlace nonwoven layer. The bonded carded nonwoven layer was formed from 6 denier polyester staple fibers. Additionally, a thermal bonded 24 gsm hydrophilic high-loft nonwoven was made from a resin formula of 70/30 to 60/40 of a first polypropylene / a second polypropylene-based copolymer. The polypropylene-based copolymer was a copolymer of polypropylene and polyethylene, with an ethylene content of about 15 wt%. In this regard, the amount of polyethylene in the high-loft nonwoven was around 4.5 wt.% - 6 wt.%. In accordance with certain embodiments of the invention, one or more of the bonded carded layers may be replaced with the thermal bonded high-loft nonwoven.

Table 1 summarizes the physical properties of each nonwoven layer. As shown in Table 1, each nonwoven layer had an absorption rate of below 4 seconds for 5 ml of liquid and demonstrated a run off percentage of zero.

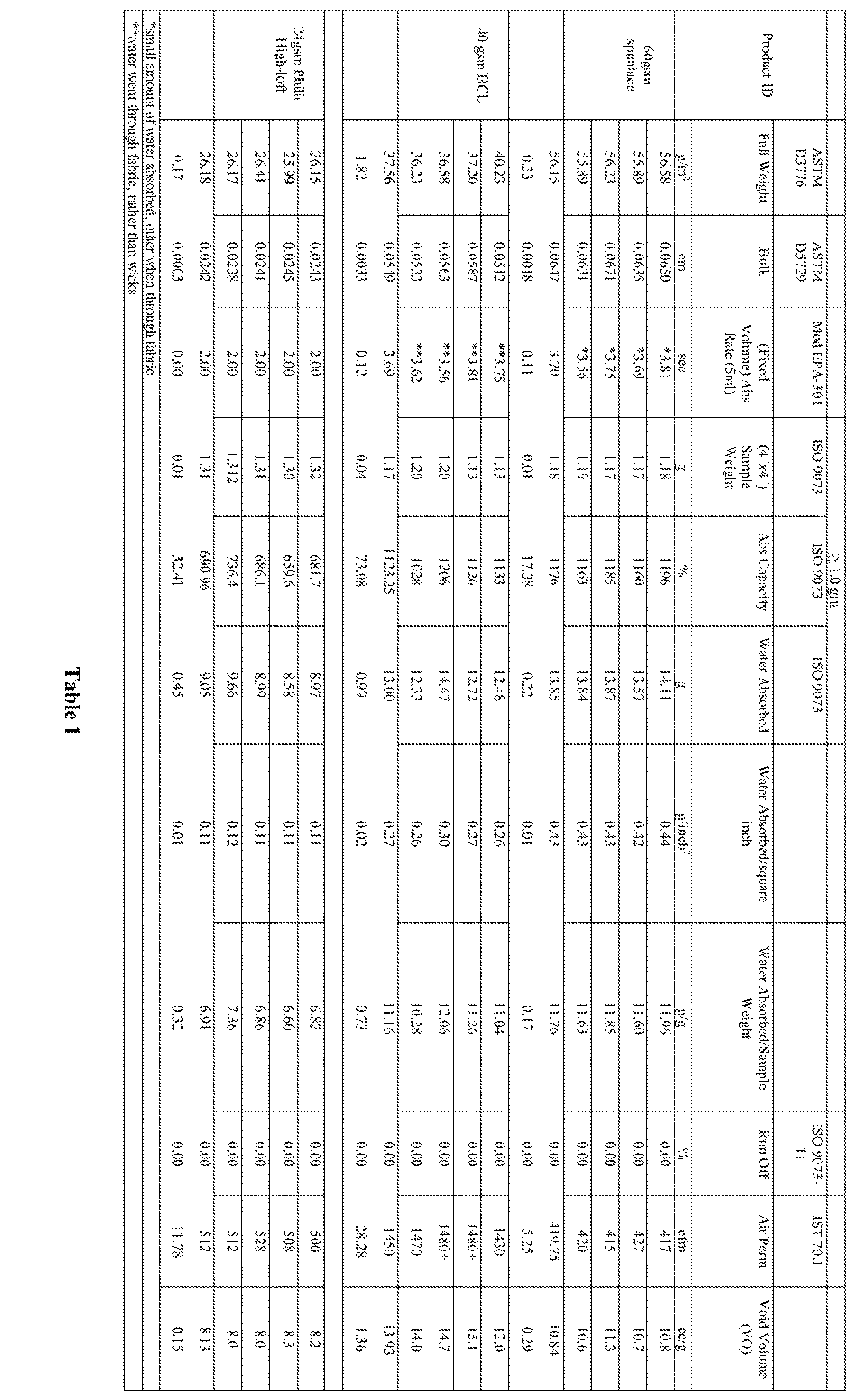

### Example Set (2)

A variety of comparative absorbent composites were obtained and tested for a variety of physical properties as well as three (3) inventive absorbent composites.

Inventive absorbent composite (1) included a 10 gsm SSMMS a top nonwoven layer of polypropylene, a first spunlace nonwoven layer of 60 gsm as described in Example Set (1), one 40 gsm bonded carded nonwoven layer as described in Example Set (1), a second spunlace nonwoven layer of 60 gsm as described in Example Set (1), and a 20 gsm film layer adhesively bonded to the second spunlace nonwoven layer with a 3 gsm adhesive layer. In this regard, the bonded carded nonwoven layer is located directly between the two spunlace nonwoven layers and the top nonwoven layer is directly bonded to the first spunlace nonwoven layer. In particular, inventive absorbent composite (1) has the following layered structure: top nonwoven layer / first spunlace nonwoven layer / bonded carded nonwoven layer / second spunlace nonwoven layer / adhesive layer / film layer. Inventive absorbent composite (2) has the same structure as inventive absorbent composite (1), but includes an additional bonded carded nonwoven layer located between the two spunlace nonwoven layers. Inventive absorbent composite (3) has the same structure as inventive absorbent composite (2), but includes a 30 gsm film instead of a 20 gsm film.

The combination of the absorbent rate and the run off percentage are of particular importance for consideration in absorbent composites. As summarized in Table 2, the absorbent composites in accordance with certain embodiments of the invention (i.e., Inventive Composites 1 - 3) each demonstrated far superior run off percentages and absorption times relative to all of the comparative absorbent composites. For example, each of the inventive absorbent composites included an absorption rate for 5 ml that was about 43% of the absorption rate for the Lambs Medical absorbent composite. Even more impressively, each of the inventive absorbent composites included a run off percentage that was at most about 30% of the run off percentage of the Lambs Medical absorbent composite, in which Inventive Composite (3) had a run off percentage below 1%.

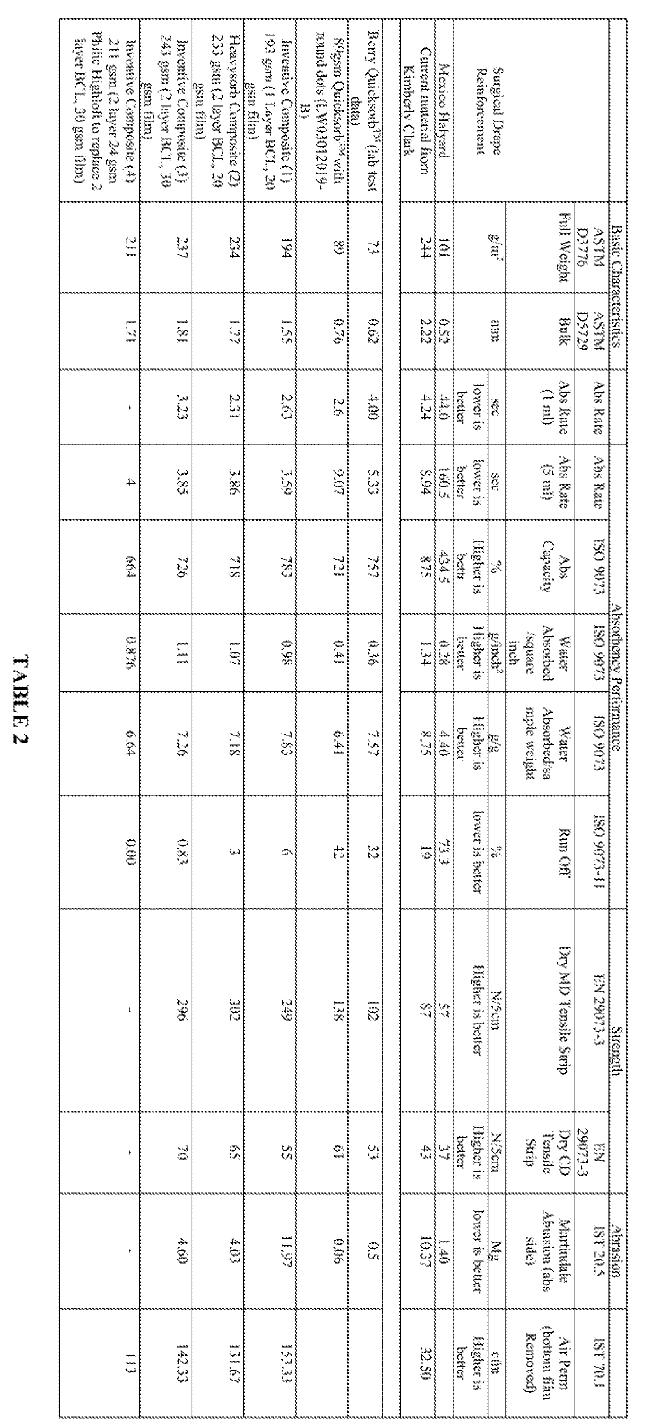

These and other modifications and variations to the invention may be practiced by those of ordinary skill in the art without departing from the scope of the invention, which is more particularly set forth in the appended claims. In addition, it should be understood that aspects of the various embodiments may be interchanged in whole or in part. Furthermore, those of ordinary skill in the art will appreciate that the foregoing description is by way of example only, and it is not intended to limit the invention as further described in such appended claims. Therefore, the scope of the appended claims should not be limited to the exemplary description of the versions contained herein.

## Claims

1. An absorbent composite, comprising:
(a) a nonwoven top layer comprising hydrophilic fibers; and
(b) an absorbent core directly or indirectly attached to the nonwoven top layer, the absorbent core comprising a plurality of layers including (i) at least one physically entangled nonwoven layer, and (ii) at least one bonded carded nonwoven layer and/or at least one thermal bonded high-loft nonwoven web;
wherein the absorbent composite includes (i) a composite-rate of absorption of less than 8 seconds for a 5 ml liquid sample as determined by D824-94, and (ii) a composite-run-off value of less than 15% as determined by ISO 9073-11.

2. The absorbent composite of claim 1, wherein the nonwoven top layer comprises a spunmelt nonwoven, such as a spunbond nonwoven, meltblown nonwoven, or a combination thereof, such as a S1ₐ-M_{b}-S2_{c} structure; wherein 'S1' is a first spunbond material, 'M' is a meltblown material, 'S2' is a second spunbond material, and 'a', 'b', and 'c' indicate the number of respective layers and each may independently be selected from 1, 2, 3, 4, or 5.

3. The absorbent composite of claims 1-2, wherein the nonwoven top layer comprises (i) at least about 30% by weight of hydrophilic fibers, such as at least about 40%, at least about 50%, at least about 60%, at least about 70%, at least about 80%, at least about 90%, or about 100% by weight of hydrophilic fibers, and (ii) an air permeability of at least about 50 cubic-feet-per-minute (CFM) as determined by IST 70.1, such as at least about 100 CFM, such as at least about 150 CFM, at least about 200 CFM, at least about 250 CFM, at least about 300 CFM, at least about 350 CFM, at least about 400 CFM, or at least about 500 CFM as determined by IST 70.1.

4. The absorbent composite of claims 1-3, wherein the nonwoven top layer includes a bonding area prior to formation of the absorbent composite comprising from about 1-30%, such at least about any of the following: 1, 3, 5, 6, 8, 10, 12, and 15%, and/or at most about any of the following: 30, 28, 25, 22, 20, 18, and 15%.

5. The absorbent composite of claims 1-4, wherein the absorbent core includes a single physically entangled nonwoven layer and a single bonded carded nonwoven layer, wherein the single physically entangled nonwoven layer is directly or indirectly located between the nonwoven top layer and the single bonded carded nonwoven layer.

6. The absorbent composite of claims 1-4, wherein the absorbent core includes a first physically entangled nonwoven layer, a second physically entangled nonwoven layer, and a first bonded carded nonwoven layer, wherein the first bonded carded nonwoven layer is directly or indirectly located between the first physically entangled nonwoven layer and the second physically entangled nonwoven layer.

7. The absorbent composite of claim 6, further comprising a second bonded carded nonwoven layer, the first bonded carded web is directly or indirectly located between the first physically entangled nonwoven layer and the second bonded carded nonwoven layer.

8. The absorbent composite of claims 1-7, wherein the first bonded carded nonwoven layer, the second bonded carded nonwoven layer, or both independently comprise a plurality of synthetic staple fibers comprising a synthetic polymeric material, such as a polyolefin, a polyester, a polyamide, or any combination thereof.

9. The absorbent composite of claim 8, wherein the first bonded carded nonwoven layer, the second bonded carded nonwoven layer, or both independently have a void volume greater than 9 cc/g, such as greater than about 12 cc/g, greater than about 14 cc/g, greater than about 15 cc/g, greater than about 16 cc/g, greater than about 17 cc/g, greater than about 18 cc/g, greater than about 19 cc/g, greater than about 20 cc/g, greater than about 25 cc/g, or greater than about 30 cc/g.

10. The absorbent composite of claims 6-9, wherein the first physically entangled nonwoven layer, the second physically entangled nonwoven layer, or both independently include from about 10% to about 90% by weight of synthetic polymeric fibers, such as at least about any of the following: 10, 15, 20, 25, 30, 35, 40, 45, and 50% by weight of synthetic polymeric fibers, and/or at most about any of the following: 90, 80, 70, 60, and 50% by weight of synthetic polymeric fibers, wherein the synthetic polymeric fibers comprise a synthetic polymer comprising polyolefin, a polyester, a polyamide, or any combination thereof.

11. The absorbent composite of claim 10, wherein the synthetic polymeric fibers comprise from about 20% to about 100% by weight of hydrophilic fibers, such as at least about any of the following: 20, 30, 40, and 50% by weight of hydrophilic fibers, and/or at most about any of the following: 100, 90, 80, 70, 60, and 50% by weight of hydrophilic fibers, wherein the hydrophilic fibers of the first physically entangled nonwoven layer, the second physically entangled nonwoven layer, or both independently comprise a hydrophilic coating thereon and/or he hydrophilic fibers of the first physically entangled nonwoven layer, the second physically entangled nonwoven layer, or both independently comprise a hydrophilic additive dispersed throughout the synthetic polymeric material.

12. The absorbent composite of claims 6-11, wherein the first physically entangled nonwoven layer, the second physically entangled nonwoven layer, or both independently include from about 10% to about 90% by weight of cellulosic fibers, such as at least about any of the following: 10, 15, 20, 25, 30, 35, 40, 45, and 50% by weight of cellulosic polymeric fibers, and/or at most about any of the following: 90, 80, 70, 60, and 50% by weight of cellulosic fibers, wherein the cellulosic fibers comprises cellulose acetate, regenerated cellulose, saponified acetate, or any combination thereof.

13. The absorbent composite of claims 6-12, wherein the first physically entangled nonwoven layer, the second physically entangled nonwoven layer, or both comprises a plurality of apertures extending through an entire thickness in the z-direction, and wherein (i) the plurality of apertures define an open area, the open area comprising from about 1% to about 30%, such as at least about any of the following: 1, 2, 3, 5, 6, 8, 10, 12, 14, and 15%, and/or at most about any of the following: 30, 28, 26, 25, 24, 22, 20, 18, 16, and 15%, and/or (ii) the plurality of apertures have an average diameter from about 0.1 mm to about 2 mm, such as at least about any of the following: 0.1, 0.2, 0.4, .05. 0.6, 0.8, and 1 mm, and/or at most about any of the following: 2, 1.8, 1.6, 1.5, 1.4, 1.2, and 1 mm.

14. A method of making an absorbent composite, comprising:
(a) providing a nonwoven top layer comprising hydrophilic fibers;
(b) providing an absorbent core, the absorbent core comprises a plurality of layers including (i) at least one physically entangled nonwoven layer, and (ii) at least one bonded carded nonwoven layer; and
(c) directly or indirectly attaching the nonwoven top layer and the absorbent core to provide the absorbent composite; wherein the absorbent composite comprises an absorbent composite according to any one of claims 1-13.

15. The absorbent composite of claims 1-13, further comprising a film layer bonded to the absorbent core, wherein the absorbent core is located directly or indirectly between the film layer and the nonwoven top layer.

## Patentansprüche

1. Absorbierender Verbundstoff, umfassend:
(a) eine Vliesstoffdeckschicht, umfassend hydrophile Fasern; und
(b) einen absorbierenden Kern, der direkt oder indirekt an der Vliesstoffdeckschicht befestigt ist, wobei der absorbierende Kern eine Vielzahl von Schichten umfasst, beinhaltend (i) mindestens eine physikalisch verschlaufte Vliesstoffschicht und (ii) mindestens eine gebundene kardierte Vliesstoffschicht und/oder mindestens eine thermisch gebundene hochflorige Vliesstoffbahn;
wobei der absorbierende Verbundstoff (i) eine Verbundstoffabsorptionsrate von weniger als 8 Sekunden für eine 5-ml-Flüssigkeitsprobe, bestimmt nach D824-94, und (ii) einen Verbundstoffablaufwert von weniger als 15 %, bestimmt nach ISO 9073-11, beinhaltet.

2. Der absorbierende Verbundstoff nach Anspruch 1, wobei die Vliesstoffdeckschicht einen Spunmeltvliesstoff, wie einen Spunbondvliesstoff, einen Meltblownvliesstoff oder eine Kombination davon umfasst, wie eine S1ₐ-M_{b}-S2_{c}-Struktur; wobei "S1" ein erstes Spinnvliesmaterial ist, "M" ein Meltblownmaterial ist, "S2" ein zweites Spinnvliesmaterial ist und "a", "b" und "c" die Anzahl der jeweiligen Schichten angeben und jeweils unabhängig voneinander aus 1, 2, 3, 4 oder 5 ausgewählt werden können.

3. Der absorbierende Verbundstoff nach den Ansprüchen 1 bis 3, wobei die Vliesstoffdeckschicht (i) mindestens etwa 30 Gew.-% hydrophile Fasern, wie etwa mindestens etwa 40 %, mindestens etwa 50 %, mindestens etwa 60 %, mindestens etwa 70 %, mindestens etwa 80 %, mindestens etwa 90 % oder etwa 100 Gew.-% hydrophile Fasern, und (ii) eine Luftdurchlässigkeit von mindestens etwa 50 Kubikfuß pro Minute (CFM), wie durch IST 70.1 bestimmt, wie mindestens etwa 100 CFM, wie mindestens etwa 150 CFM, mindestens etwa 200 CFM, mindestens etwa 250 CFM, mindestens etwa 300 CFM, mindestens etwa 350 CFM, mindestens etwa 400 CFM oder mindestens etwa 500 CFM, wie durch IST 70.1 bestimmt, umfasst.

4. Der absorbierende Verbundstoff nach den Ansprüchen 1 bis 3, wobei die Vliesstoffdeckschicht eine Bindungsfläche vor der Bildung des absorbierenden Verbundstoffs beinhaltet, umfassend etwa 1 bis 30 %, wie mindestens etwa eine der folgenden: 1, 3, 5, 6, 8, 10, 12 und 15%, und/oder höchstens etwa eine der folgenden: 30, 28, 25, 22, 20, 18 und 15 %.

5. Der absorbierende Verbundstoff nach den Ansprüchen 1 bis 4, wobei der absorbierende Kern eine einzelne physikalisch verschlaufte Vliesstoffschicht und eine einzelne gebundene kardierte Vliesstoffschicht beinhaltet, wobei die einzelne physikalisch verschlaufte Vliesstoffschicht direkt oder indirekt zwischen der Vliesstoffdeckschicht und der einzelnen gebundenen kardierten Vliesstoffschicht angeordnet ist.

6. Der absorbierende Verbundstoff nach den Ansprüchen 1 bis 4, wobei der absorbierende Kern eine erste physikalisch verschlaufte Vliesstoffschicht, eine zweite physikalisch verschlaufte Vliesstoffschicht und eine erste gebundene kardierte Vliesstoffschicht umfasst, wobei die erste gebundene kardierte Vliesstoffschicht direkt oder indirekt zwischen der ersten physikalisch verschlauften Vliesstoffschicht und der zweiten physikalisch verschlauften Vliesstoffschicht angeordnet ist.

7. Der absorbierende Verbundstoff nach Anspruch 6, weiter umfassend eine zweite gebundene kardierte Vliesstoffschicht, wobei die erste gebundene kardierte Bahn direkt oder indirekt zwischen der ersten physikalisch verschlauften Vliesstoffschicht und der zweiten gebundenen kardierten Vliesstoffschicht angeordnet ist.

8. Der absorbierende Verbundstoff nach den Ansprüchen 1 bis 7, wobei die erste gebundene kardierte Vliesstoffschicht, die zweite gebundene kardierte Vliesstoffschicht oder beide unabhängig voneinander eine Vielzahl synthetischer Stapelfasern umfassen, umfassend ein synthetisches Polymermaterial, wie ein Polyolefin, einen Polyester, ein Polyamid oder irgendeine Kombination davon.

9. Der absorbierende Verbundstoff nach Anspruch 8, wobei die erste gebundene kardierte Vliesstoffschicht, die zweite gebundene kardierte Vliesstoffschicht oder beide unabhängig voneinander ein Hohlraumvolumen von mehr als 9 cc/g, wie beispielsweise mehr als etwa 12 cc/g, mehr als etwa 14 cc/g, mehr als etwa 15 cc/g, mehr als etwa 16 cc/g, mehr als etwa 17 cc/g, mehr als etwa 18 cc/g, mehr als etwa 19 cc/g, mehr als etwa 20 cc/g, mehr als etwa 25 cc/g oder mehr als etwa 30 cc/g, besitzen.

10. Der absorbierende Verbundstoff nach den Ansprüchen 6 bis 9, wobei die erste physikalisch verschlaufte Vliesstoffschicht, die zweite physikalisch verschlaufte Vliesstoffschicht oder beide unabhängig voneinander etwa 10 bis etwa 90 Gew.-% synthetische Polymerfasern beinhalten, wie mindestens etwa eine der folgenden: 10, 15, 20, 25, 30, 35, 40, 45 und 50 Gew.-% synthetische Polymerfasern und/oder höchstens etwa eine der folgenden: 90, 80, 70, 60 und 50 Gew.-% synthetische Polymerfasern, wobei die synthetischen Polymerfasern ein synthetisches Polymer umfassen, umfassend ein Polyolefin, einen Polyester, ein Polyamid oder irgendeine Kombination davon.

11. Der absorbierende Verbundstoff nach Anspruch 10, wobei die synthetischen Polymerfasern etwa 20 bis etwa 100 Gew.-% hydrophile Fasern umfassen, wie mindestens etwa eine der folgenden: 20, 30, 40 und 50 Gew.-% hydrophile Fasern, und/oder höchstens etwa eine der folgenden: 100, 90, 80, 70, 60 und 50 Gew.-% hydrophile Fasern, wobei die hydrophilen Fasern der ersten physikalisch verschlauften Vliesschicht, der zweiten physikalisch verschlauften Vliesschicht oder beider unabhängig voneinander eine hydrophile Beschichtung darauf umfassen und/oder die hydrophilen Fasern der ersten physikalisch verschlauften Vliesschicht, der zweiten physikalisch verschlauften Vliesschicht oder beider unabhängig voneinander ein hydrophiles Additiv umfassen, das in dem synthetischen Polymermaterial dispergiert ist.

12. Der absorbierende Verbundstoff nach den Ansprüchen 6 bis 11, wobei die erste physikalisch verschlaufte Vliesschicht, die zweite physikalisch verschlaufte Vliesschicht oder beide unabhängig voneinander etwa 10 bis etwa 90 Gew.-% Cellulosefasern, wie mindestens etwa eine der folgenden, beinhalten: 10, 15, 20, 25, 30, 35, 40, 45 und 50 Gew.-% cellulosische Polymerfasern und/oder höchstens etwa eine der folgenden: 90, 80, 70, 60 und 50 Gew.-% cellulosische Fasern, wobei die cellulosischen Fasern Celluloseacetat, regenerierte Cellulose, verseiftes Acetat oder irgendeine Kombination davon umfassen.

13. Der absorbierende Verbundstoff nach den Ansprüchen 6 bis 12, wobei die erste physikalisch verschlaufte Vliesschicht, die zweite physikalisch verschlaufte Vliesschicht oder beide eine Vielzahl von Öffnungen umfassen, die sich durch eine gesamte Dicke in der z-Richtung erstrecken, und wobei (i) die Vielzahl von Öffnungen einen offenen Bereich definieren, wobei der offene Bereich etwa 1 % bis etwa 30 % umfasst, wie beispielsweise mindestens etwa eines der folgenden: 1, 2, 3, 5, 6, 8, 10, 12, 14 und 15 %, und/oder höchstens etwa eine der folgenden: 30, 28, 26, 25, 24, 22, 20, 18, 16 und 15 %, und/oder (ii) die Vielzahl von Öffnungen einen durchschnittlichen Durchmesser von etwa 0,1 mm bis etwa 2 mm, wie mindestens etwa einen der folgenden, besitzen: 0.1, 0.2, 0.4, .05. 0,6, 0,8 und 1 mm, und/oder höchstens etwa einen der folgenden: 2, 1,8, 1,6, 1,5, 1,4, 1,2, und 1 mm.

14. Verfahren zur Herstellung eines absorbierenden Verbundstoffs, umfassend:
(a) Bereitstellen einer Vliesstoffdeckschicht, umfassend hydrophile Fasern;
(b) Bereitstellen eines absorbierenden Kerns, wobei der absorbierende Kern eine Vielzahl von Schichten umfasst, beinhaltend (i) mindestens eine physikalisch verschlaufte Vliesstoffschicht und (ii) mindestens eine gebundene kardierte Vliesstoffschicht; und
(c) direktes oder indirektes Befestigen der Vliesstoffdeckschicht und des absorbierenden Kerns, um den absorbierenden Verbundstoff bereitzustellen; wobei der absorbierende Verbundstoff einen absorbierenden Verbundstoff nach irgendeinem der Ansprüche 1 bis 13 umfasst.

15. Der absorbierende Verbundstoff nach den Ansprüchen 1 bis 13, weiter umfassend eine an den saugfähigen Kern gebundene Filmschicht, wobei der saugfähige Kern direkt oder indirekt zwischen der Filmschicht und der Vliesstoffdeckschicht angeordnet ist.

## Revendications

1. Composite absorbant, comprenant :
(a) une couche supérieure non-tissée comprenant des fibres hydrophiles ; et
(b) un cœur absorbant directement ou indirectement fixé à la couche supérieure non-tissée, le cœur absorbant comprenant une pluralité de couches incluant (i) au moins une couche non-tissée physiquement enchevêtrée, et (ii) au moins une couche non-tissée cardée-liée et/ou au moins un voile non-tissé à pouvoir gonflant élevé, thermiquement lié ;
dans lequel le composite absorbant inclut (i) un taux d'absorption du composite inférieur à 8 secondes pour un échantillon de liquide de 5 ml tel que déterminé selon D824-94, et (ii) une valeur d'égouttement du composite inférieure à 15 % telle que déterminée selon la norme ISO 9073-11.

2. Composite absorbant selon la revendication 1, dans lequel la couche supérieure non-tissée comprend un non-tissé filé à l'état fondu, tel qu'un non-tissé filé-lié, un non-tissé de fusion-soufflage, ou une combinaison de ceux-ci, telle qu'une structure S1ₐ-M_{b}-S2_{c} ; dans laquelle « S1 » est un premier matériau filé-lié, « M » est un matériau de fusion-soufflage, « S2 » est un second matériau filé-lié, et « a », « b », et « c » indiquent le nombre de couches respectives et peuvent chacun indépendamment être sélectionnés parmi 1, 2, 3, 4, ou 5.

3. Composite absorbant selon les revendications 1 à 2, dans lequel la couche supérieure non-tissée comprend (i) au moins environ 30 % en poids de fibres hydrophiles, tel que d'au moins environ 40 %, d'au moins environ 50 %, d'au moins environ 60 %, d'au moins environ 70 %, d'au moins environ 80 %, d'au moins environ 90 %, ou environ 100 % en poids de fibres hydrophiles, et (ii) une perméabilité à l'air d'au moins environ 50 pieds cubiques par minute (CFM) tel que déterminé selon la norme IST 70.1, tel que d'au moins environ 100 CFM, tel que d'au moins environ 150 CFM, d'au moins environ 200 CFM, d'au moins environ 250 CFM, d'au moins environ 300 CFM, d'au moins environ 350 CFM, d'au moins environ 400 CFM, ou d'au moins environ 500 CFM tel que déterminé selon la norme IST 70.1.

4. Composite absorbant selon les revendications 1 à 3, dans lequel la couche supérieure non-tissée inclut une zone de liaison avant la formation du composite absorbant comprenant d'environ 1 à 30 %, tel que d'au moins environ l'un quelconque de ce qui suit : 1, 3, 5, 6, 8, 10, 12, et 15 %, et/ou d'au plus environ l'un quelconque de ce qui suit : 30, 28, 25, 22, 20, 18, et 15 %.

5. Composite absorbant selon les revendications 1 à 4, dans lequel le cœur absorbant inclut une couche non-tissée unique physiquement enchevêtrée et une couche non-tissée unique cardée-liée, dans lequel la couche non-tissée unique physiquement enchevêtrée est directement ou indirectement localisée entre la couche supérieure non-tissée et la couche non-tissée unique cardée-liée.

6. Composite absorbant selon les revendications 1 à 4, dans lequel le cœur absorbant inclut une première couche non-tissée physiquement enchevêtrée, une seconde couche non-tissée physiquement enchevêtrée, et une première couche non-tissée cardée-liée, dans lequel la première couche non-tissée cardée-liée est directement ou indirectement localisée entre la première couche non-tissée physiquement enchevêtrée et la seconde couche non-tissée physiquement enchevêtrée.

7. Composite absorbant selon la revendication 6, comprenant en outre une seconde couche non-tissée cardée-liée, le premier voile cardé-lié est directement ou indirectement localisé entre la première couche non-tissée physiquement enchevêtrée et la seconde couche non-tissée cardée-liée.

8. Composite absorbant selon les revendications 1 à 7, dans lequel la première couche non-tissée cardée-liée, la seconde couche non-tissée cardée-liée, ou toutes deux indépendamment comprennent une pluralité de fibres discontinues synthétiques comprenant un matériau polymère synthétique, tel qu'une polyoléfine, un polyester, un polyamide, ou une quelconque combinaison de ceux-ci.

9. Composite absorbant selon la revendication 8, dans lequel la première couche non-tissée cardée-liée, la seconde couche non-tissée cardée-liée, ou toutes les deux indépendamment présentent un volume de vide supérieur à 9 cc/g, tel que supérieur à environ 12 cc/g, supérieur à environ 14 cc/g, supérieur à environ 15 cc/g, supérieur à environ 16 cc/g, supérieur à environ 17 cc/g, supérieur à environ 18 cc/g, supérieur à environ 19 cc/g, supérieur à environ 20 cc/g, supérieur à environ 25 cc/g, ou supérieur à environ 30 cc/g.

10. Composite absorbant selon les revendications 6 à 9, dans lequel la première couche non-tissée physiquement enchevêtrée, la seconde couche non-tissée physiquement enchevêtrée, ou toutes les deux indépendamment incluent d'environ 10 % à environ 90 % en poids de fibres polymères synthétiques, tel que d'au moins environ l'un quelconque de ce qui suit : 10, 15, 20, 25, 30, 35, 40, 45, et 50 % en poids de fibres polymères synthétiques, et/ou d'au plus environ l'un quelconque de ce qui suit : 90, 80, 70, 60, et 50 % en poids de fibres polymères synthétiques, dans lequel les fibres polymères synthétiques comprennent un polymère synthétique comprenant une polyoléfine, un polyester, un polyamide, ou une quelconque combinaison de ceux-ci.

11. Composite absorbant selon la revendication 10, dans lequel les fibres polymères synthétiques comprennent d'environ 20 % à environ 100 % en poids de fibres hydrophiles, tel que d'au moins environ l'un quelconque de ce qui suit : 20, 30, 40, et 50 % en poids de fibres hydrophiles, et/ou d'au plus environ l'un quelconque de ce qui suit : 100, 90, 80, 70, 60, et 50 % en poids de fibres hydrophiles, dans lequel les fibres hydrophiles de la première couche non-tissée physiquement enchevêtrée, de la seconde couche non-tissée physiquement enchevêtrée, ou toutes les deux indépendamment comprennent un revêtement hydrophile dessus et/ou les fibres hydrophiles de la première couche non-tissée physiquement enchevêtrée, de la seconde couche non-tissée physiquement enchevêtrée, ou toutes les deux indépendamment comprennent un additif hydrophile dispersé à travers le matériau polymère synthétique.

12. Composite absorbant selon les revendications 6 à 11, dans lequel la première couche non-tissée physiquement enchevêtrée, la seconde couche non-tissée physiquement enchevêtrée, ou toutes les deux indépendamment incluent d'environ 10 % à environ 90 % en poids de fibres cellulosiques, tel que d'au moins environ l'un quelconque de ce qui suit : 10, 15, 20, 25, 30, 35, 40, 45, et 50 % en poids de fibres polymères cellulosiques, et/ou d'au plus environ l'un quelconque de ce qui suit : 90, 80, 70, 60, et 50 % en poids de fibres cellulosiques, dans lequel les fibres cellulosiques comprennent de l'acétate de cellulose, de la cellulose régénérée, de l'acétate saponifié, ou une quelconque combinaison de ceux-ci.

13. Composite absorbant selon les revendications 6 à 12, dans lequel la première couche non-tissée physiquement enchevêtrée, la seconde couche non-tissée physiquement enchevêtrée, ou toutes deux comprennent une pluralité d'ouvertures s'étendant à travers une épaisseur entière dans le sens z, et dans lequel (i) la pluralité d'ouvertures définissent une zone ouverte, la zone ouverte comprenant d'environ 1 % à environ 30 %, tel que d'au moins environ l'un quelconque de ce qui suit : 1, 2, 3, 5, 6, 8, 10, 12, 14, et 15 %, et/ou d'au plus environ l'un quelconque de ce qui suit : 30, 28, 26, 25, 24, 22, 20, 18, 16, et 15 %, et/ou (ii) la pluralité d'ouvertures présentent un diamètre moyen d'environ 0,1 mm à environ 2 mm, tel que d'au moins environ l'un quelconque de ce qui suit : 0, 1, 0,2, 0,4, 0,5, 0,6, 0,8, et 1 mm, et/ou d'au plus environ l'un quelconque de ce qui suit : 2, 1,8, 1, 6, 1,5, 1,4, 1,2, et 1 mm.

14. Procédé de fabrication d'un composite absorbant, comprenant :
(a) la fourniture d'une couche supérieure non-tissée comprenant des fibres hydrophiles ;
(b) la fourniture d'un cœur absorbant, le cœur absorbant comprend une pluralité de couches incluant (i) au moins une couche non-tissée physiquement enchevêtrée, et (ii) au moins une couche non-tissée cardée-liée ; et
(c) la fixation directement ou indirectement de la couche supérieure non-tissée et du cœur absorbant pour fournir le composite absorbant ; dans lequel le composite absorbant comprend un composite absorbant selon l'une quelconque des revendications 1 à 13.

15. Composite absorbant selon les revendications 1 à 13, comprenant en outre une couche formant film liée au cœur absorbant, dans lequel le cœur absorbant est localisé directement ou indirectement entre la couche formant film et la couche supérieure non-tissée.
